(19) Europäisches Patentamt
European Patent Office
Office européen des brevets

(11) Publication number: **0 119 040 B1**

(12) **EUROPEAN PATENT SPECIFICATION**

(45) Date of publication of patent specification: **27.05.87**

(51) Int. Cl.$^4$: **C 12 P 33/06,** C 07 J 41/00, C 07 J 9/00

(21) Application number: **84301378.0**

(22) Date of filing: **02.03.84**

(54) **Producing conjugated ursodeoxycholic acids.**

(30) Priority: **04.03.83 JP 35554/83**

(43) Date of publication of application:
**19.09.84 Bulletin 84/38**

(45) Publication of the grant of the patent:
**27.05.87 Bulletin 87/22**

(84) Designated Contracting States:
**AT BE CH DE FR GB IT LI LU NL SE**

(56) References cited:
**EP-A-0 088 637**

**APPLIED AND ENVIRONMENTAL MICROBIOLOGY, vol. 44, no. 6, December 1982, pages 1249-1252, American Society for Microbiology, Washington, D.C., US H. SAWADA et al.: "Microbial production of ursodeoxycholic acid from lithocholic acid by Fusarium equiseti M41"**

(73) Proprietor: **KABUSHIKI KAISHA YAKULT HONSHA**
**1-19, Higashishinbashi 1-chome**
**Minato-ku Tokyo 105 (JP)**

(72) Inventor: **Sawada, Haruji**
**16-8, 3-chome Fujimidai Kunitachi**
**Tokyo (JP)**
Inventor: **Watanuki, Masaaki**
**1364-81 Nakashin-cho Akishima**
**Tokyo (JP)**

(74) Representative: **Brewer, Leonard Stuart et al**
**Sanderson & Co. 97 High Street**
**Colchester Essex CO1 1TH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 119 040 B1**

## Description

The present invention relates to a method for producing conjugated ursodeoxycholic acids by means of microbial transformation, and more specifically to a method for producing one or more ursodeoxycholic acids conjugated with one or more amino acids (hereinafter referred to as conjugated ursodeoxycholic acids) which comprises subjecting one or more lithocholic acids conjugated with one or more amino acids (hereinafter referred to as conjugated lithocholic acids) to the action of one or more microorganisms having the capability of producing conjugated ursodeoxycholic acids.

## BACKGROUND OF THE INVENTION

Conjugated ursodeoxycholic acids, the final products of a method according to the invention, have the molecular structure shown in formula (I):

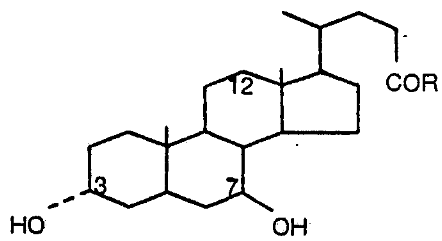

(I)

wherein R is such that RH represents an amino acid. Referring to this formula, a conjugated ursodeoxycholic acid is a bile acid wherein hydroxyl groups are located at 3α- and 7β-positions thereof and the carboxyl group located in a side chain thereof is conjugated with an amino acid (including artificially produced amino acids).

Out of the conjugated ursodeoxycholic acids, tauroursodeoxycholic acid, ursodeoxycholic acid conjugated with taurine, is the major constituent of the gall-bladder of a bear. This compound is a potential raw material for producing ursodeoxycholic acid which has recently been employed as a cholagogue or a cholesterol solubilizer. In other words, ursodeoxycholic acid can be produced by deconjugation of tauroursodeoxycholic acid. Since the supply of good gall-bladders of bears is fairly limited, it is difficult to satisfy the entire medical demands from the aforementioned natural supply. This is the reason why chemical synthesis processes have been employed for producing ursodeoxycholic acid.

However, each of the chemical synthesis processes available in the prior art involves a plurality of complicated steps. Since the number of steps each of which is fairly complex, is sometimes as large as 7, the prior art processes have the drawbacks that the process time is long, the process is complicated, the efficiency is inferior due to the purification processes needed after each individual step, and the yield is unsatisfactory due to side reactions which inevitably accompany each individual step.

To try to overcome these drawbacks, various one step methods have been proposed for producing ursodeoxycholic acid by means of microbial transformation wherein a β-hydroxyl group is introduced to be bonded with the carbon atom at the 7-position of the steroid nucleus (ring) of lithocholic acid.

We succeeded in inventing a one step method for producing free ursodeoxycholic acid from free lithocholic acid, which comprises subjecting lithocholic acid to the action of any of the specific microorganisms or moulds belonging to *Fusarium*.

However, the transformational reaction substrate for this one step method for producing ursodeoxycholic acid is free lithocholic acid which does not necessarily readily dissolve in a cultivation medium and/or a reaction medium. Accordingly, the hydrophobic nature of the transformational reaction substrate readily causes a lower degree of microbial transformation in the aforementioned method, resulting in a lower yield, a lower reaction rate and lower productivity.

In view of the fact that free ursodeoxycholic acid is converted, in the human body, to conjugated ursodeoxycholic acids, when it is internally applied, we assumed that some conjugated ursodeoxycholic acids would have medicinal value which is equivalent or superior to that of free ursodeoxycholic acid. Based on this assumption, and also based on the fact that conjugated lithocholic acids readily dissolve in water, we thought that producing one or more conjugated ursodeoxycholic acids by means of microbial transformation which comprises subjecting one or more conjugated lithocholic acids to the action of one or more microorganisms which have the capability of producing conjugated ursodeoxycholic acids, would overcome the aforementioned drawbacks.

## OBJECTS AND SUMMARY OF THE INVENTION

An object of the present invention is to provide a one step method for producing conjugated ursodeoxycholic acids from conjugated lithocholic acids by means of a microbial transformation technique, which overcomes the aforementioned drawbacks such as lower yield, lower reaction rate and lower productivity.

The invention provides a method for producing ursodeoxycholic acid conjugated with an amino acid, which conjugated ursodeoxycholic acid has the formula (I):

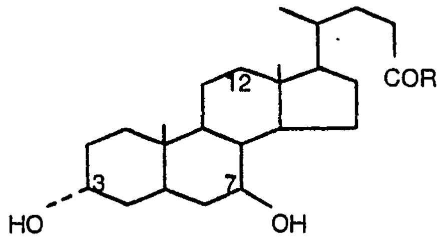

(I)

wherein R is such that RH represents an amino acid, which method comprises subjecting lithochloic acid conjugated with an amino acid, which conjugated lithocholic acid has the formula (II):

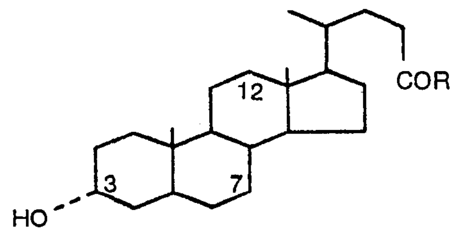

(II)

wherein R is as defined above, to the action of a microorganism belonging to *Mortierella* which is capable of transforming the conjugated lithocholic acid to the conjugated ursodeoxycholic acid.

The invention provides also a process for preparing ursodeoxycholic acid, which process comprises deconjugating conjugated ursodeoxycholic acid produced by this method. The deconjugating can be performed in a way known *per se*.

The invention also provides *per se* a microorganism of particular use in the present method of production, viz the microorganism *Mortierella ramanniana* var. *ramanniana* strain Y2—1 whose Deposition number is FERM BP—440. As found in nature the microorganism tends not to be suitable for industrial use. It may be extracted from nature in ways which are conventional in themselves. We have discovered this particular microorganism and its valuable property of transforming conjugated lithocholic acid to conjugated ursodeoxycholic acid. The microorganism should be extracted to a conventional extent to make it suitable for cultivation. The microorganism of the invention is usually that produced by cultivation rather than directly extracted from nature.

The method according to the present invention for producing a conjugated ursodeoxycholic acid from a conjugated lithocholic acid comprises subjecting a conjugated lithocholic acid to the action of any of the microorganisms or moulds belonging to *Mortierella*, whereby a β-hydroxyl group is introduced to be bonded with the carbon atom at the 7-position of the steroid nucleus (ring) of the conjugated lithocholic acid. Conjugated ursodeoxycholic acid produced is then usually recovered.

More specifically, the method according to the invention for producing a conjugated ursodeoxycholic acid comprises a step of bringing a conjugated lithocholic acid into contact with any part or parts of any of the microorganisms or moulds which have the capability of producing conjugated ursodeoxycholic acids from conjugated lithocholic acids and which belong to *Mortierella*, typically *Mortierella ramanniana* var. *ramanniana* strain Y2—1 (hereinafter referred to as strain Y2—1) in any manner (e.g. in any type of reaction liquid, such as a nutrient medium, a reaction medium *et al.*).

Particularly, the present method is for producing ursodeoxycholic acid conjugated with an amino acid by microbial transformation of lithocholic acid conjugated with the amino acid, comprising:

(A) a step of cultivating a microorganism belonging to *Mortierella* which is capable of transforming the conjugated lithocholic acid to the conjugated ursodeoxycholic acid;

(B) a step of bringing the conjugated lithocholic acid into contact with the microorganism; and

(C) a step of recovering conjugated ursodeoxycholic acid produced.

Preferred embodiments of the present method are:

(1) A method for producing a conjugated ursodeoxycholic acid from a conjugated lithocholic acid, wherein the step of bringing the conjugated lithocholic acid into contact with the microorganism occurs in a medium in which the microorganism is cultivated, and which further includes a step of recovering conjugated ursodeoxycholic acid.

(2) A method for producing a conjugated ursodeoxycholic acid from a conjugated lithocholic acid, (i) which further comprises a first preparatory step in which the microorganism is cultivated, preferably in a basal medium which readily allows good growth for the microorganism, a second preparatory step in which the microorganism is harvested, and a third preparatory step in which a reaction medium containing the harvested microorganism is prepared, (ii) in which the step of bringing the conjugated lithocholic acid into contact with the microorganism occurs in this reaction medium, and (iii) which further comprises a finishing step in which the conjugated ursodeoxycholic acid is recovered.

(3) A method for producing a conjugated ursodeoxycholic acid from a conjugated lithocholic acid, (i) which further comprises a first preparatory step in which the microorganism is cultivated, usually in an

agar medium, typically in a potato agar medium or a malt extract agar medium, until spores are formed, a second preparatory step in which the spores formed are collected, and a third preparatory step in which a reaction medium which is a suspension containing the collected spores and the conjugated lithocholic acid is prepared, (ii) in which the step of bringing the conjugated lithocholic acid into contact with the microorganism occurs in this reaction medium, and (iii) which further comprises a finishing step in which the conjugated ursodeoxycholic acid is recovered.

In embodiment (1), the conjugated lithocholic acid can be added to the medium at various phases of cultivation. The conjugated lithocholic acid can be included in a medium which is then inoculated with the microorganism, and the microorganism cultivated therein. Alternatively, the conjugated lithocholic acid can be admixed with a medium which has already been inoculated with the microorganism and in which the microorganism has been cultivated, and the cultivation then continued.

Harvested microorganism, for example in embodiment (2), can be stabilized with polyacrylamide, calcium arginate or the like, before it is brought into contact with the conjugated lithocholic acid. Harvested microorganism, for example in embodiment (2), is usually brought into contact with the conjugated lithocholic acid without delay after harvesting.

A reaction medium in which the microbial transformation occurs, for instance that in embodiment (2) or (3), preferably contains a small quantity of energy source specifically some organic materials typically glucose, some other hydrocarbons, casein hydrolysate, yeast extract or the like.

When spores of the microorganism are employed, for instance in embodiment (3), the collected spores are generally once stabilized before they are brought into contact with the conjugated lithocholic acid.

Since the conjugated lithocholic acid readily dissolves in water, the present invention provides a one step method for producing a conjugated ursodeoxycholic acid from a conjugated lithocholic acid by microbial transformation, whose yield, reaction rate and productivity are high.

In addition, the conjugated lithocholic acid, which is the starting material for the microbial transformation which is the subject of the method according to the present invention, has a unique molecular structure wherein the side chain which is bonded with the amino acid effectively covers the external surface of the molecule except for the carbon atom which is located at the 7-position of the steroid nucleus (ring), thereby readily allowing access for a β-hydroxyl group to the aforementioned 7-position of the steroid nucleus (ring) but prohibiting a hydroxyl group from approaching the other positions of the steroid nucleus (ring), thus protecting the molecule from any possibility of side reactions. Due to this molecular structure of the conjugated lithocholic acid, the present method for producing a conjugated ursodeoxycholic acid can proceed with an excellent yield, which can be as high as 95%.

Another advantage of the present method is that a large quantity of any conjugated lithocholic acid can be made readily available, because it can be produced readily, for instance by mixing free lithocholic acid with one or more specific amino acids at a low temperature in dioxane containing tributylamine and ethyl chlorocarbonate, thus enabling the productivity to be increased for the method in accordance with the present invention. Although some free lithocholic acid remains unconjugated with the amino acid during the aforementioned reaction, this remaining free lithocholic acid causes absolutely no adverse effects for the microbial transformation.

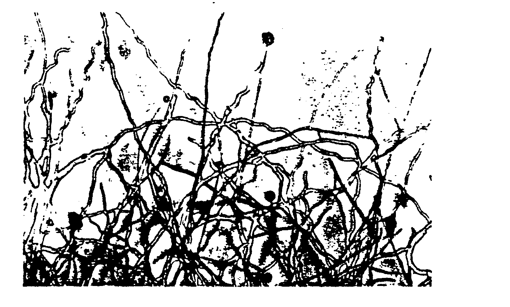

## BRIEF DESCRIPTION OF THE DRAWING

The present invention, together with its various features and advantages, can be readily understood from the following more detailed description presented in conjunction with the accompanying drawing.

The sole figure of the drawing is a reproduction of a micro-photograph showing the appearance of strain Y2—1, a microorganism belonging to *Mortierella ramanniana* var. *ramanniana*, which is an example of a strain which has the capability of producing conjugated ursodeoxycholic acids from conjugated lithocholic acids.

## DETAILED DESCRIPTION

(A) Identification of Strain Y2—1

In the middle phase of cultivation of strain Y2—1 carried out at 27°C on a malt extract agar medium, sporangia which confine many spores therein, grow at the end of sporangiophores which are 300—700 µm long. The sporangiophores grow on the side of a mycelia in the form of single extension or in the form of branched extension.

A small columella is recognized at the top end of the sporangiophore inside the opended sporangium in the last phase of cultivation. The spores have an oval shape, and their longer diameter varies between 3 µm and 5.5 µm and their shorter diameter varies between 1.5 µm and 2.5 µm.

The chlamydospores have various shapes, including spherical, and many oil drops are recognized inside them.

No zygospores are recognized. This microorganism has a tendency to require thiamine.

Five-day cultivation carried out on a melt extract agar medium allows strain Y2—1 to grow up to a colony whose diameter ranges from 2.4 to 2.7 cm. A colony whose thickness ranges from 2 to 3 mm is velvety and vinaceous-brown.

The sole figure of the accompanying drawing is a reproduction of a micro-photograph showing the appearance of strain Y2—1 which has been cultivated at 27°C for 1 week on a malt extract agar medium

comprising 20 grams of malt extract, 1 gram of peptone, 20 grams of glucose, 20 grams of agar and 1 (one) litre of water and having a pH value of 6.5. The magnification in the drawing is 150 when the figure is 11 by 7.2 cm in size.

In accordance with the classification of Gams *et al.* (Gams W., Domsch K. H., Anderson Traute-Heidi, Compendium of Soil Fungi: Academic Press, London, New York, Toronto, Sydney, San Francisco (1980)), strain Y2—1 described above was identified as *Mortierella ramanniana* var. *ramanniana* and named *Mortierella ramanniana* var. *ramanniana* Y2—1. This is because the appearance of strain Y2—1 is identical to the reference appearance of *Mortierella ramanniana* and the strain requires thiamine. Strain Y2—1 has been deposited at the Fermentation Research Institute, Agency of Industrial Science & Technology, Japan with the Deposition number FERM BP—440.

The physiological properties of strain Y2—1 are as follows:

(1) Optimum growth conditions (pH and temperature). The optimum pH range is 6 to 7, and the optimum temperature range is 27 to 28°C.

(2) Allowable growth conditions (pH and temperature). The allowable pH range is 3 to 8, and the allowable temperature range is 5 to 38°C.

(3) Other specific feature

Thiamine requirement is recognized.

(B) Screening of Strain Y2—1

Numerous mould strains were isolated from soil samples picked up in and adjacent to the Tama district of Tokyo, Japan. Each of these mould strains was inoculated into 100 ml of a screening medium kept in a 500 ml Sakaguchi-flask, and was incubated on a shaker at 27°C for 7 days. After completion of the incubation, the pH value of the culture (20 ml) was adjusted to 3 with 5N hydrochloric acid, before conjugated bile acids were extracted with 50 ml of n-butanol. After being dehydrated with Glauber's salt, the extract was vacuum-condensed by means of a rotary evaporator. Qualitative analysis was applied to each of the condensed extracts by means of thin layer chromatography, in order to screen any strain which is capable of producing conjugated ursodeoxycholic acids from conjugated lithocholic acids. In the aforementioned thin layer chromatography, the bands for the condensed extract were compared with those for the corresponding conjugated ursodeoxycholic acids.

In this manner, we were successful in screening a strain which is capable of producing conjugated ursodeoxycholic acids from conjugated lithocholic acids. The screened strain was named strain Y2—1. The mould strain from which we were successful in screening strain Y2—1 was isolated from the soil picked up at a field located in Yaho Kunitachi, Tokyo on July 14, 1982.

The basal medium employed for isolation of strain Y2—1 comprised 1 (one) litre of water, 50 g of glucose, 1 g of $KH_2PO_4$, 2 g of $K_2HPO_4$, 0.5 g of $MgSO_4.7H_2O$, 5 g of polypeptone, 2 g of yeast extract, 10 mg of $CaCl_2$, 10 mg of $FeSO_4.7H_2O$ and 0.5 g of conjugated lithocholic acid which was the transformational reaction substrate.

(C) Example 1

A liquid medium is prepared by mixing 1 (one) litre of water with 50 g of glucose, 5 g of polypeptone, 2 g of yeast extract, 2 g of $K_2HPO_4$, 1 g of $KH_2PO_4$, 0.5 g of $MgSO_4.7H_2O$, 10 mg of $CaCl_2$, 10 mg of $FeSO_4.7H_2O$, 10 mg of thiamine and 0.5 g of taurolithocholic acid. This liquid medium is employed to cultivate strain Y2—1 on a shaker at 27°C for 48 hours in a Sakaguchi-flask. 6 litres of the aforementioned liquid medium is poured into a jar fermentor having a capacity of 10 litres. Strain Y2—1 is inoculated in the liquid medium contained in the jar fermentor in an amount of 5%, before it is cultivated under aerobic conditions at 27°C for 5 days. During the cultivation, the agitation speed is kept at 150 rpm, the pH value is adjusted to 6, and air is supplied at a rate of 0.5 vvm (air volume per liquid volume per minute). After completion of the cultivation, 5N NaOH is added to the culture to adjust the pH value to 7. Thereafter, the cultivated microorganisms are collected out of the culture. The collected microorganisms are washed with water. A mixture of the washing liquid and the filtered liquid remaining after the microorganisms have been collected from the culture, is passed through a column filled with 500 g of a porous resin (Amberlite XAD—2), to allow the column to absorb the mixture of the conjugated ursodeoxycholic acid produced and the conjugated lithocholic acid which has remained unchanged during the transformation process.

After the column which has absorbed the mixture has been washed with water, methanol is employed to elute out the conjugated ursodeoxycholic acid and the conjugated lithocholic acid. This methanol fraction containing tauroursodeoxycholic acid is condensed by means of a rotary evaporator. This condensed methanol fraction is applied to a column filled with 200 g of silica gel (Wakogel C—200), before there is applied to the column a mixture of solvents composed of isoamyl alcohol, acetic acid and water at the volumetric ratio of 18:5:3 to elute tauroursodeoxycholic acid. The elution is split into a plurality of fractions. Out of these fractions, one or more fractions containing much tauroursodeoxycholic acid is or are selectively collected. In this manner, tauroursodeoxycholic acid can be separated from taurolithocholic acid which is the transformational reaction substrate. A solvent containing ethanol and ethyl acetate is employed to crystallize tauroursodeoxycholic acid contained in the collected fraction or fractions.

The yield of crystallized tauroursodeoxycholic acid is 0.49 g from 1 (one) litre of reaction medium, representing 95% in terms of mol equivalent in comparison with the raw material.

(D) Example 2

A liquid medium is prepared by mixing 1 (one) litre of water with 50 g of glucose, 5 g of polypeptone, 2 g of yeast extract, 1 g of $KH_2PO_4$, 2 g of $K_2HPO_4$, 0.5 g of $MgSO_4.7H_2O$, 10 mg of $CaCl_2$, 10 mg of $FeSO_4.7H_2O$, 10 mg of thiamine and 0.5 g of glycolithocholic acid which is the transformational reaction substrate. This liquid medium is employed to incubate strain Y2—1 on a shaker at 27°C for 48 hours in a Sakaguchi-flask. 6 litres of the aforementioned liquid medium is poured into a jar fermentor having a capacity of 10 litres. Strain Y2—1 is inoculated in the liquid medium contained in the jar fermentor in an amount of 5%, before it is cultivated under aerobic conditions at 27°C for 5 days. During the cultivation, the agitation speed is kept at 150 rpm, the pH value is adjusted to 6, and air is supplied at a rate of 0.5 vvm.

After completion of the cultivation, 5N hydrochloric acid is added to the culture to adjust the pH value to 3. Thereafter, the conjugated bile acids are extracted with 18 litres of n-butanol. This extract is condensed by means of a rotary evaporator.

As is in the case of Example 1, silica gel column chromatography is employed to purify the condensed extract and to elute one or more fractions, this time containing glycoursodeoxycholic acid. Thereafter, the glycoursodeoxycholic acid contained in the fraction is crystallized from a solvent mixture of ethanol and ethyl acetate. The yield of crystallized glycoursodeoxycholic acid is 0.48 g from 1 (one) litre of reaction medium, representing 93% in terms of mol equivalent in comparison with the raw material.

Separately, the condensed extract is dissolved in 15% NaOH solution, and a deconjugation process known in the prior art is carried out at 120°C for 5 hours. Free ursodeoxycholic acid is thereby separated from glycine, though both stay in the deconjugated condensed extract. The free ursodeoxycholic acid is extracted with ethyl acetate. The ethyl acetate containing free ursodeoxycholic acid is dried by means of Glauber's salt, before being condensed by means of a rotary evaporator.

This condensed material is applied to a column filled with 80 g of silica gel (Wakogel C—200), before there is applied to the column a mixture of solvents composed of chloroform, acetone and acetic acid at the volumetric ratio of 100:100:1 to elute free ursodeoxycholic acid. The elution is split into a plurality of fractions. Out of these fractions, one or more fractions containing much free ursodeoxycholic acid is or are selectively collected. After removing the solvent, the fractions containing free ursodeoxycholic acid have added to them a small volume of ethyl acetate, and thereby free ursodeoxycholic acid is crystallized.

The yield of crystallized free ursodeoxycholic acid is 0.42 g from 1 (one) litre of reaction medium, representing 93% in term of mol equivalent in comparison with the raw material.

(E) Example 3

A liquid medium is prepared by mixing 1 (one) litre of water with 50 g of glucose, 5 g of polypeptone, 2 g of yeast extract, 1 g of $KH_2PO_4$, 2 g of $K_2HPO_4$, 0.5 g of $MgSO_4.7H_2O$, 10 mg of $CaCl_2$, 10 mg of $FeSO_4.7H_2O$ and 10 mg of thiamine. This liquid medium is employed to cultivate strain Y2—1 on a shaker at 27°C for 48 hours in a Sakaguchi-flask. 6 litres of the aforementioned liquid medium is poured into a jar fermentor having a capacity of 10 litres. Strain Y2—1 is inoculated in the liquid medium contained in the jar fermentor in an amount of 5%, before it is cultivated under aerobic conditions at 27°C for 108 hours. When 36 hours have passed after commencement of the cultivation, 3 g of lithocholic acid conjugated with sarcosine is poured into the jar fermentor. During the cultivation, the agitation speed is kept at 150 rpm, the pH is adjusted to 6, and air is supplied at a rate of 0.5 vvm.

A series of isolation processes identical to those employed in Example 1 is applied to the culture, to collect ursodeoxycholic acid conjugated with sarcosine in a yield of 0.48 g per 1 (one) litre of reaction medium, representing 93% in terms of mol equivalent in comparison with the raw material.

(F) Example 4

Strain Y2—1 is cultivated for 48 hours in a medium which is identical to that employed in Example 1 except that it does not contain taurolithocholic acid. The cultivated microorganisms are collected by means of a filter. After the collected microorganisms have been washed with a phosphoric acid buffer having a pH value of 7.0, they are added to a reaction medium comprising 1 g of $KH_2PO_4$, 2 g of $K_2HPO_4$, 10 g of glucose, 0.5 g of yeast extract, 0.2 g of glycolithocholic acid and 1 (one) litre of water and which has a pH value of 7.0, thereby forming a suspension containing 20 g of the microorganisms in 1 (one) litre of the reaction medium. In this suspension, a transformational reaction is conducted under aerobic conditions at 27°C for 48 hours.

The later processes for collection of crystallized glycoursodeoxycholic acid are identical to those employed in Example 1.

The yield is 0.19 g from 1 (one) litre of reaction medium, representing 92% in terms of mol equivalent in comparison with the raw material.

(G) Example 5

Strain Y2—1 is inoculated on a malt extract agar medium containing 1 (one) litre of water with 20 g of malt extract, 1 g of polypeptone and 20 g of agar and which has a pH value of 7.0, and is cultivated at 27°C for 1 week under aerobic conditions.

After completion of the cultivation, the cultivated microorganisms are collected and are added to a

phosphoric acid buffer having a pH value of 7.0. The buffer is filtered with a gauze filter to collect spores of the microorganism, and the collected spores are washed with water before being added to a phosphoric acid buffer having a pH value of 7.0. The quantity of the spores in the reaction medium is adjusted to be $10^{10}$ spores/litre.

The reaction medium has added to it 5 g/litre of glucose, 0.2 g/litre of yeast extract and 0.2 g/litre of taurolithocholic acid, before a transformational reaction is conducted at 27°C for 72 hours under conditions where sterilized air is supplied.

The later processes for collection of crystallized tauroursodeoxycholic acid are identical to those employed in Example 1.

The yield is 0.19 g from 1 (one) litre of transformational reaction medium, representing 92% in terms of mol equivalent in comparison with the raw material.

(H) Identification of conjugated ursodeoxycholic acid produced with reference conjugated ursodeoxycholic acid

Firstly, thin layer chromatography (Merck Kieselgel G—60, $F_{254}$, 0.25 mm thick), utilizing development solvents for the analysis of bile acids, was employed to identify each of the products of Examples 1 to 5 with the corresponding standard conjugated ursodeoxycholic acids (produced and supplied by Techno Chemical Co., Ltd.). The $R_f$ values determined for each of the products of Examples 1 to 5 are identical to the $R_f$ values determined for the corresponding standard conjugated ursodeoxycholic acid.

Additionally, the various analytical techniques listed below were applied to each of the products of Examples 1 to 5 to identify each of them with the aforementioned standard conjugated ursodeoxycholic acids.

1. Elementary analysis
2. Melting point test and mixed melting point test
3. Infrared spectrum analysis
4. Mass spectrum analysis
5. Nuclear magnetic resonsance spectrum analysis
6. Analysis employing equipment (manufactured by Nihon Bunko Kogyo Co., Ltd.) for high performance liquid chromatography in which a column immobilized with 3α-hydroxysteroid dehydrogenase is employed for analysis of bile acids.

The results of all the foregoing tests determined that the properties of each of the products of Examples 1 to 5 are identical to those of the corresponding standard conjugated ursodeoxycholic acid.

(I) Conclusion

The foregoing description shows that a one step method for producing conjugated ursodeoxycholic acids from conjugated lithocholic acids by microbial transformation has successfully been provided by the present invention and that this method has overcome the drawbacks in the aforementioned one step method for producing free ursodeoxycholic acid from free lithocholic acid by microbial transformation, namely a lower yield, a lower reaction rate and lower productivity. In other words, the method of the present invention has various features including a higher yield, a higher reaction rate and higher productivity.

In the present method, the conjugatged amino acid can be, for example, taurine, glycine or sarcosine.

Although the present invention has been described with reference to a specific strain, strain Y2—1 whose Deposition number is FERM BP—440, this is not meant to be construed in a limiting sense. Various modifications of the specific embodiments, including using other microorganisms or moulds belonging to *Mortierella*, will be apparent to persons skilled in the art upon reference to the description of the present invention.

**Claims**

1. A method for producing ursodeoxycholic acid conjugated with an amino acid, which conjugated ursodeoxycholic acid has the formula (I):

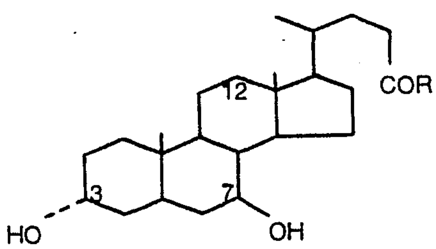

(I)

wherein R is such that RH represents an amino acid, which method comprises subjecting lithocholic acid conjugated with an amino acid, which conjugated lithocholic acid has the formula (II):

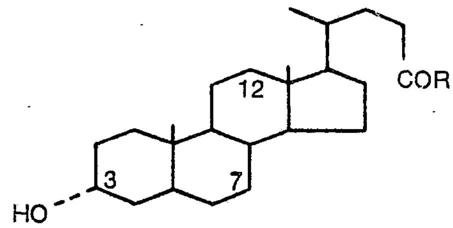

(II)

wherein R is as defined above, to the action of a microorganism belonging to *Mortierella* which is capable of transforming the conjugated lithocholic acid to the conjugated ursodeoxycholic acid.

2. A method for producing ursodeoxycholic acid conjugated with an amino acid by microbial transformation of lithocholic acid conjugated with the amino acid, comprising:

(A) a step of cultivating a microorganism belonging to *Mortierella* which is capable of transforming the conjugated lithocholic acid to the conjugated ursodeoxycholic acid;

(B) a step.of bringing the conjugated lithocholic acid into contact with the microorganism; and

(C) a step of recovering conjugated ursodeoxycholic acid produced.

3. A method according to claim 2, wherein step (B) of bringing the conjugated lithocholic acid into contact with the microorganism occurs in a medium in which step (A) of cultivating the microorganism is carried out.

4. A method according to claim 2, (a) which further comprises a step of harvesting the microorganism and a step of preparing a reaction medium which is a suspension containing the harvested microorganism, and (b) in which step (B) of bringing the conjugated lithocholic acid into contact with the microorganism occurs in this reaction medium.

5. A method according to claim 2, (a) in which step (A) of cultivating the microorganism is carried out until spores are formed, (b) which further comprises a step of harvesting the spores and a step of preparing a reaction medium which is a suspension containing the harvested spores, and (c) in which step (B) of bringing the conjugated lithocholic acid into contact with the microorganism occurs in this reaction medium.

6. A method according to any one of the preceding claims, wherein the microorganism is *Mortierella ramanniana* var. *ramanniana* strain Y2—1 whose Deposition number is FERM BP—440.

7. A method according to any one of the preceding claims, wherein the amino acid is taurine, glycine or sarcosine.

8. A process for preparing ursodeoxycholic acid, which process comprises deconjugating conjugated ursodeoxycholic acid produced by a method claimed in any one of the preceding claims.

9. The microorganism *Mortierella ramanniana* var. *ramanniana* strain Y2—1 whose Deposition number is FERM BP—440.

**Patentansprüche**

1. Eine Methode zur Erzeugung von Ursodeoxycholsäure, die mit einer Aminosäure konjugiert ist, wobei die konjugierte Ursodeoxycholsäure die Formel (I)

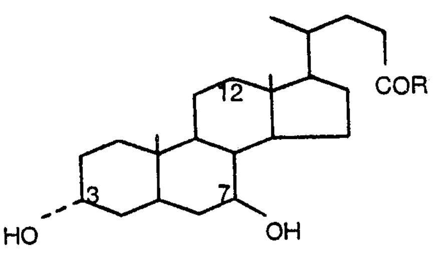

(I)

hat, in welcher R so beschaffen ist, daß RH eine Aminosäure darstellt, und diese Methode die Aussetzung einer mit Aminosäure konjugierten Lithocholsäure nach Formel (II),

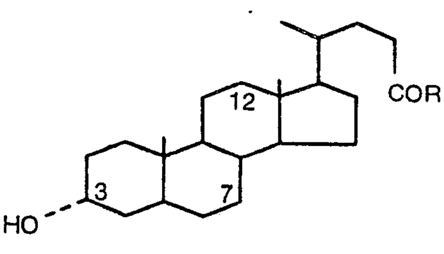

(II)

in der R der obigen Definition entspricht, der Wirkung von Mikroorganismen der Familie *Mortierella* beinhaltet, die konjugierte Lithocholsäure in konjugierte Ursodeoxycholsäure umwandeln können.

2. Eine Methode zur Erzeugung von Ursodeoxycholsäure, die mittels mikrobischer Transformation einer mit Aminosäure konjugierten Lithocholsäure mit Aminosäure konjugiert ist, wobei die Methode

(A) einen Schritt zur Züchtung von Mikroorganismen der Familie *Mortierella* mit der Fähigkeit einer Transformation der konjugierten Lithocholsäure in konjugierte Ursodeoxycholsäure;

(B) einen Schritt zur Inkontaktbringung der konjugierten Lithocholsäure mit den Mikroorganismen und

(C) einen Schritt zur Gewinnung der erzeugten konjugierten Ursodeoxycholsäure einschließt.

3. Eine Methode nach Patentanspruch 2 in welcher der Schritt (B) mit der Inkontaktbringung der konjugierten Lithocholsäure mit den Mikroorganismen in einem Mittel erfolgt, in welchem der Schritt (A) zur Züchtung der Mikroorganismen durchgeführt wird.

4. Eine Methode nach Patentanspruch 2 die (a) zusätzlich einen Schritt zur Erntung der Mikroorganismen und einen Schritt zur Bereitung eines die geernteten Mikroorganismen in Suspension enthaltenen Reaktionsmittels umfaßt, und in der (b) der Schritt (B) mit der Inkontaktbringung der konjugierten Lithocholsäure mit den Mikroorganismen in diesem Reaktionsmittel erfolgt.

5. Eine Methode nach Patentanspruch 2 in der (a) der Schritt (A) mit der Züchtung von Mikroorganismen bis zur Sporenbildung durchgeführt wird, (b) die Methode einen weiteren Schritt zur Erntung der Sporen und einen Schritt zur Bereitung eines die geernteten Sporen in Suspension enthaltenen Reaktionsmittels umfaßt, und (c) der Schritt (B) mit der Inkontaktbringung der konjugierten Lithocholsäure mit den Mikroorganismen in diesem Reaktionsmittel erfolgt.

6. Eine Methode nach irgendeinem der vorhergehenden Patentansprüche wobei die Mikroorganismen zur Familie *Mortierella ramannina* Abart *ramannina* Stamm Y2—1 mit der Kennzeichnungsnummer FERM BP—440 gehören.

7. Eine Methode nach irgendeinem der vorhergehenden Patentansprüche wobei die Aminosäure Taurin, Glyzin oder Sarkosin ist.

8. Ein Verfahren zur Bereitung von Ursodeoxycholsaüre wobei dieses Verfahren entkonjugierte konjugierte Ursodeoxycholsäure verwendet, die mittels einer Methode nach irgendeinem der vorhergehenden Patentansprüche hergestellt wurde.

9. Mikroorganismen der Familie *Mortierella ramanniana* Abart *ramanniana* Stamm Y2—1 mit der Kennzeichnungsnummer FERM BP—440.

**Revendications**

1. Une méthode permettant de produire de l'acide ursodéoxycholique conjugué avec un acide aminé, lequel acide ursodéoxycholique conjugué comporte la formule (I):

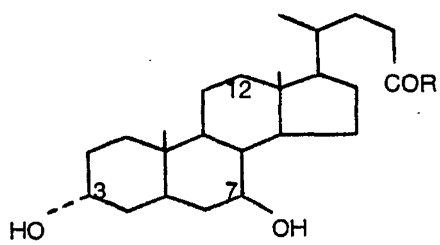

(I)

où R est tel que RH représente un acide aminé. Cette méthode consiste à soumettre de l'acide lithocholique conjugué avec un acide aminé — lequel acide lithocholique possède la formule (II):

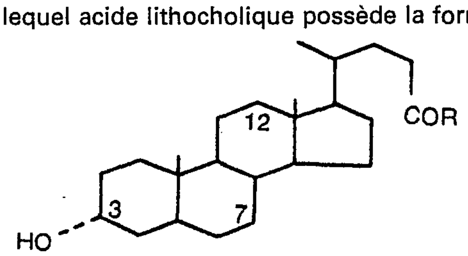

(II)

où R est défini comme ci-dessus — à l'action d'un microorganisme appartenant à *Mortierella*, capable de transformer l'acide lithocholique en acide ursodéoxycholique conjugué.

2. Une méthode permettant de produire de l'acide ursodéoxycholique conjugué avec un acide aminé par transformation microbienne d'acide lithocholique conjugué avec l'acide aminé, comprenant les étapes suivantes:

(A) la culture d'un micro-organisme appartenant à *Mortierella*, capable de transformer l'acide lithocholique conjugué en acide ursodéoxycholique conjugué;

(B) la mise en contact de l'acide lithocholique conjugué avec le micro-organisme; et

(C) la récupération de l'acide ursodéoxycholique conjugué ainsi produit.

3. Une méthode, selon la revendication 2, dans laquelle l'étape (B) (mise en contact de l'acide lithocholique conjugé avec le micro-organisme) s'effectue dans un milieu de culture dans lequel l'étape (A) (culture du micro-organisme) a été exécutée.

4. Une méthode, selon la revendication 2, a) comprenant en outre, une étape consistant à récolter le micro-organisme et une étape consistant à preparer un milieu de culture de réaction, celui-ci étant une suspension contenant le micro-organisme récolté, et b) dans laquelle l'étape (B) (mise en contact de l'acide lithocholique conjugué avec le micro-organisme) s'effectue dans ce milieu de réaction.

5. Une méthode, selon la revendication 2, a) dans laquelle l'étape (A) (culture du micro-organisme) est exécutée jusqu'à ce que des spores soient formés, et b) qui, en outre, comprend une étape consistant à récolter les spores et une étape consistant à préparer un milieu de culture de réaction, celui-ci étant une suspension contenant les spores récoltés, et c) dans laquelle l'étape (B) (mise en contact de l'acide lithocholique avec le micro-organisme) s'effectue dans ce milieu de réaction.

6. Une méthode, selon n'importe laquelle des revendications précédentes, dans laquelle le micro-organisme se nomme *Mortierella ramanniana* var. *ramanniana* souche Y2—1, dont le numéro de dépot est BP—440.

7. Une méthode, selon n'importe laquelle des revendications précédentes, dans laquelle l'acide aminé est la taurine, la glycine ou la sarcosine.

8. Un procédé pour la préparation d'acide ursodéoxycholique, consistant à déconjuguer l'acide ursodéoxycholique conjugué, produit selon n'importe quelle méthode revendiquée dans les revendications précédentes.

9. Le micro-organisme *Mortierella ramanniana* var. *ramanniana* souche Y2—1, numéro de dépot FERM BP—440.